Europäisches Patentamt

European Patent Office

Office européen des brevets

(19)

(11) Publication number:

**0 301 667**
**A1**

# (12) EUROPEAN PATENT APPLICATION

(21) Application number: **88201622.3**

(22) Date of filing: **27.07.88**

(51) Int. Cl.⁴: **G01N 33/92 , G01N 33/535**

The title of the invention has been amended (Guidelines for Examination in the EPO, A-III, 7.3).

(30) Priority: **28.07.87 GB 8717791**

(43) Date of publication of application:
**01.02.89 Bulletin 89/05**

(84) Designated Contracting States:
**AT BE CH DE ES FR GB IT LI LU NL SE**

(71) Applicant: **ISTITUTO SIEROTERAPICO MILANESE "S. BELFANTI"**
**Via Darwin, 22**
**I-20143 Milano(IT)**

(72) Inventor: **Baralle, Francisco Ernesto**
**Residenza Acacie**
**I-20080 Basiglio Milano 3 Milan(IT)**
Inventor: **Sidoli, Alessandro**
**Via Friuli 75**
**I-20135 Milan(IT)**

(74) Representative: **Appoloni, Romano et al**
**Ing. Barzanò & Zanardo S.p.A. Via Borgonuovo 10**
**I-20121 Milano(IT)**

(54) **NEW METHOD OF IMMUNOASSAY**

(57) A method of detection or estimation of an apolipoprotein in a sample which comprises contacting a solid support having an antibody to said apolipoprotein bound thereto with the sample containing said apolipoprotein and with fused protein as defined herein comprising at least an antigenic part of said apolipoprotein and a label protein and then observing or measuring the label protein either bound or not bound to the said support.

EP 0 301 667 A1

## NEW METHOD OF IMMUNOASSAY

This invention relates to methods of immunoassay for the detection or estimation of an apolipoprotein, in particular the determination of plasma apolipoprotein levels.

Immunoassay methods are widely used for the determination of proteins in samples of body fluids. For such methods to be satisfactory, a number of requirements must be fulfilled:

(a) A primary standard of the protein to be assayed must be obtained. Frequently such proteins are not very stable, e.g. because they form aggregates, and this adversely affects their reliability as standards. Moreover, with only a few exceptions, the standard sample must be obtained by laborious separation methods, e.g. plasma fractionation.

(b) Proteins are usually assayed by immunological methods. In consequence the specificity of the assay depends upon the specificity of the antigen-antibody reaction. Such specificity can be improved by use of monoclonal antibodies.

(c) The immunoassay procedure adopted must be sufficiently sensitive to detect the required protein at the levels at which it actually occurs. In some cases, e.g. with apolipoproteins, the difference between normal and pathological levels is not very great. Immunoassay methods which depend upon use of radioactive isotopes are among the most sensitive methods currently in use. However, the handling of radioisotopes requires use of special equipment because of safety considerations and is in general to be avoided. Immunoenzymatic methods (e.g. ELISA and EMIT) are therefore preferred when they are sufficiently sensitive.

(d) The immunoassay method must be sufficiently reproducible to be reliable. As already noted, this depends in part on the stability of the primary standard. It also depends upon the other parameters of the experimental procedure.

(e) An immunoassay method which is to be widely used must be simple and cheap to operate. Expensive reagents such as purified plasma proteins obtained by fractionation of human plasma are therefore to be avoided whenever possible. The reagents used should be stable, easy to store, and should not present any problems in disposal. No risk to the operator should be involved.

(f) Methods which are suitable for automatic operation possess clear advantages where wide scale use is desirable, e.g. in routine screening of samples from patients.

As already noted, the present invention is of particular interest in the measurement of plasma apolipoprotein levels. In order to assess predisposition to cardiovascular disease believed to be associated with hyperlipidemia, it is desirable to be able to measure not only plasma cholesterol and triglycerides, but also the fractions of cholesterol associated with low density lipoproteins (e.g. apolipoprotein B), and high density apolipoproteins (e.g. apolipoprotein AI). Recent studies have suggested that the ratio of the plasma level of apolipoprotein AI to apolipoprotein B is a more specific and sensitive indicator of cardiovascular risk than the absolute values of the cholesterol and triglyceride levels. The method of the present invention is particularly well suited to the determination of this ratio in a simple and rapid way.

The method of the present invention is based on the use of a fused (or hybrid) protein produced by recombinant DNA technology which includes both sequences from the alipoprotein to be measured and a label protein. It is known to use DNA technology to produce a gene which codes sequentially for two proteins. WO 87/02061 discloses a drug delivery system in which a peptide active ingredient and a carrier for the active ingredient are covalently bound to a low density lipoprotein (LDL) receptor binding region such as that of apolipoprotein B or apolipoprotein E. The delivery system is expressed from DNA which codes for the LDL receptor binding region and the peptide active ingredient.

WO 86/06742 discloses the use in an enzyme immunoassay of a fused protein which comprises the label enzyme $\beta$-galactosidase and a protein such as human surfactant apoprotein, human atrial natriuretic factor or the hemagglutin protein contained within influenza virus. However the gene which codes for the protein is produced entirely synthetically from oligonucleotides and the sequence produced only contains e.g. 12 or 29 amino acids i.e. only a small fragment of the protein. Such a sequence does not enable the assay to be carried out with sufficient precision in the case of very large proteins such as apolipoproteins since the enzyme labelled protein fragment and the protein to be assayed do not sufficiently resemble one another. This problem is complicated by the fact that the assay is not carried out on a simple solution of the protein to be assayed; rather the material to be assayed is a clinical sample such as human serum which contains a multitude of other substances which may interfere with the assay.

Moreover, if the full sequence of a protein is not known and it is therefore not possible to construct from oligonucleotides a gene coding for an antigenic fragment of such a protein which sufficiently resembles the naturally occuring protein to enable a reliable assay to be carried out on human serum.

It is not necessary for the hybrid protein to include the whole of the protein to be detected so long as it contains at least enough of the protein to react reliably with an antibody, normally a monoclonal antibody, specific to that protein. This genetically engineered hybrid protein is used in the present invention in an immunoassay procedure in which the hybrid protein competes with the protein to be detected for an antibody bound to a solid support. The amount of the label protein bound to the support is in inverse correlation with the amount of the protein to be detected. The general principles behind carrying out immunoassay procedures in this way are well known. The present invention lies more particularly in the use of the hybrid protein which provides a simple and reliable means for conjugating the protein to be detected to the label protein in a way which does not interfere with either the capacity of the protein to bind to the antibody or the capacity of the label protein to function as such, e.g. as an enzyme producing a directly observable effect such as generation of a colour.

The present invention accordingly provides a method for the detection or estimation of an apolipoprotein in a sample which comprises contacting a solid support having an antibody to said apolipoprotein bound thereto with the sample containing said apolipoprotein and with a fused (or hybrid) protein comprising at least an antigenic part of said apolipoprotein and a label protein and then observing or measuring the label protein either bound or not bound to the said support. As explained above, the term "fused protein" or "hybrid protein" as used herein means a protein produced by expression of a gene containing a DNA sequence coding for (1) at least an antigenic sequence of the apolipoprotein to be detected and which is isolated from cDNA of the apolipoprotein and (2) a label protein. The term "label protein" means a protein capable of producing an observable effect, preferably an effect which can be quantitatively measured, so as to provide a measure of the amount of the hybrid protein which is bound to the solid support via the antibody (or alternatively the amount of the hybrid protein which has not become bound to the solid support).

The method of the present invention is normally carried out by allowing the protein in the sample and the hybrid protein to compete for the antibody bound to the support, but it is possible, and within the scope of the invention, to allow the protein in the sample first to bind to the antibody on the support, and then to measure the amount of antibody which has not conjugated to the said protein by a reaction with the fused protein. This alternative procedure clearly depends upon the amount of antibody bound to the support being more than sufficient to react with all the protein present in the sample. Normally, the competitive assay procedure is preferred.

The invention involves producing the hybrid protein by recombinant DNA technology. The corresponding DNA sequence of several apolipoproteins such as apo AI is already known. Even, however, in those cases such as apolipoprotein D where the structure of the protein is still unknown, it is nevertheless possible to make use of the invention by cloning the gene which codes for the protein and incorporating it in a plasmid so that it can be expressed in an appropriate microorganism using standard experimental techniques (e.g. as described by Maniatis, Fritsch and Saambrooke, "Molecular Cloning, a laboratory manual", Cold Spring Harbour Laboratory (1982)). A suitable method for preparing a fused protein is described in more detail below.

The hybrid protein also includes a sequence for the label protein, e.g. an enzyme or other protein the presence of which can be readily detected or measured, e.g. by fluorescence, luminescence, laser dyes etc. In general, the same proteins can be used in the present invention as has been used as labels in the immunoassay procedures of the prior art. Labels which may be used include $\beta$-galactosidase, phosphatases and peroxidases.

Using standard genetic engineering techniques, it is possible to isolate and characterize DNA sequences coding for any given protein, and thus, in the present case, both the protein to be detected and the label protein, and to construct a hybrid between the two genes (DNA sequences) coding for the two proteins. Usually the hybrid contains a bacterial gene coding for the label protein and a eukaryotic gene coding for the protein to be detected, but it is also possible to construct a hybrid containing two different bacterial genes or two different eukaryotic genes. In the first case, the expression of the eukaryotic gene is usually under the control of the bacterial promoter, but the reverse situation is also possible. In the case where both sequences are of eukaryotic origin, the hybrid may be expressed, for example, in an appropriate cell line. In each case, the promoter which controls the hybrid DNA sequence can be activated to produce a hybrid mRNA and hence a protein in which the bacterial and eukaryotic sequences (or two bacterial or two eukaryotic sequences) are fused.

The use of hybrid proteins made in this way confers important advantages on the method of the present invention. More particularly, in the preferred case in which a bacterial gene is fused to a eukaryotic gene, a fused protein containing a significant bacterial sequence is less likely to be degraded by bacterial proteases. The hybrid gene may be constructed to contain most of the signals required for bacterial gene expression and may thus be efficiently transcribed and translated. The purification procedure used on the fused protein may be simplified by making use of the biochemical characteristics of the bacterial part of the fused protein. Finally, and most significantly, the fused protein possesses in substantially unaltered form both the antigenic and other properties of the protein to be detected and also the enzymatic (or other label) activity of the label protein.

More specifically, a hybrid betagalactosidase-apolipoprotein has been produced which combines the enzymatic activity of betagalactosidase and the antigenic and hydrophilic-hydrophobic properties of apolipoprotein AI or apolipoprotein B. The betagalactosidase activity can be used in an assay procedure for the measurement of apolipoprotein levels in samples of, e.g., serum, plasma or whole blood. The apolipoprotein preserves its ability to react specifically with monoclonal antibodies directed specifically against the apolipoprotein. Use of monoclonal antibodies is preferred since in this way all variability caused by background cross reactions and product variability involved in the use of polyclonal antibodies may be eliminated.

A preferred manner of operating the method of the present invention is in general terms as follows. The monoclonal antibody is first adsorbed onto a suitable solid support, e.g. the well of a microtitre plate or a suitable plastics material, a polysaccharide matrix, paper, or Cellogel. A competition reaction for the adsorbed monoclonal antibody is then carried out using an excess of the hybrid protein and a conveniently diluted sample of the plasma, serum, or whole blood which is being examined. After removal of unbound reagents, an enzymatic reaction is started between the betagalactosidase which is bound to the solid support and a chromogen substrate specific thereto. Conveniently, the enzymatic reaction produces a colour which can be measured spectrophotometrically, e.g. at 405nm. There is then a quantitative inverse correlation between the found betagalactosidase activity and the apolipoprotein concentration in the sample.

The monoclonal antibody may be immobilized on coated ELISA wells, coated beads of, for example, Sepharose, agarose or a suitable graft co-polymer, on a coated column or membrane of, for example, a polyamide, nitrocellulose or cellulose acetate.

It is possible to carry out multiple determinations using a single solid support, e.g. a single well in a microtitre plate. For example, such wells can be coated with monoclonal antibodies specific against both apo-AI and apoB respectively and the competition reaction then carried out simultaneously using two hybrid proteins, one incorporating apoAI linked to an enzyme and the second incorporating apoB linked to a different enzyme. The two enzymes are chosen so as to be capable of producing colour forming reactions which can be measured at different wave lengths, i.e. so that the two competition reactions can be separately measured. If desired, one enzyme may bring about a colour forming reaction and the other a reaction of a different kind, e.g. the production of a fluorescent product.

The invention includes within scope a test kit for operation of the new method comprising a solid support having an antibody, preferably a monoclonal antibody, bound thereto and the hybrid protein characteristic of the invention. The usual reagents for carrying out dilutions, washes, etc., may also be incorporated.

In more detail the operation of the new method is as follows.

## Construction of the plasmid pURAI

The plasmid pURAI codes for a hybrid protein consisting of $\beta$-galactosidase followed by the complete amino acid sequence of mature human apolipoprotein AI. The ApoAI sequence was obtained from cDNA clone (Sharpe et al. Nucl. Acids Res. (1984) pp 3917-3932), from which the sequence coding for the signal peptide was removed. The sequence was inserted in the expression vector pUR291 (Ruther et al, EMBO J. (1984) 2, pp. 1791-4), which codes for the active $\beta$-galactosidase. The apoAI sequence was inserted in the correct frame as a continuation of the sequence coding for the $\beta$-galactosidase polypeptide. The plasmid pURAI is thus capable when grown in E.coli of producing a hybrid protein with the $\beta$-gal enzymatic activity and the apoAI antigenic characteristics.

## Expression and purification of the hybrid protein $\beta$-galactosidase-apolipoprotein AI

Following standard protocols for the growth of bacteria and induction of the $\beta$-gal promoter, the hybrid $\beta$-gal apoAI protein was produced (see Lorenzetti et al, FEBS Lett. (1986) 194, pp 343-6). The hybrid protein was extracted from the bacterial pellet and fractionated following standard protocols (Marston, Biochem. J. 240, 1-12 (1986); Cheng, Biochem. Biophys. Res. Commun. 111 104-111(1983); and Schoemaker et al EMBO J. 4, 775-780 (1985). The recovered β-gal activity was $1.45 \times 10^7$ units/ml of sonic extract.

Further plasmids which code for hybrid proteins were produced in the same way as pURAI.

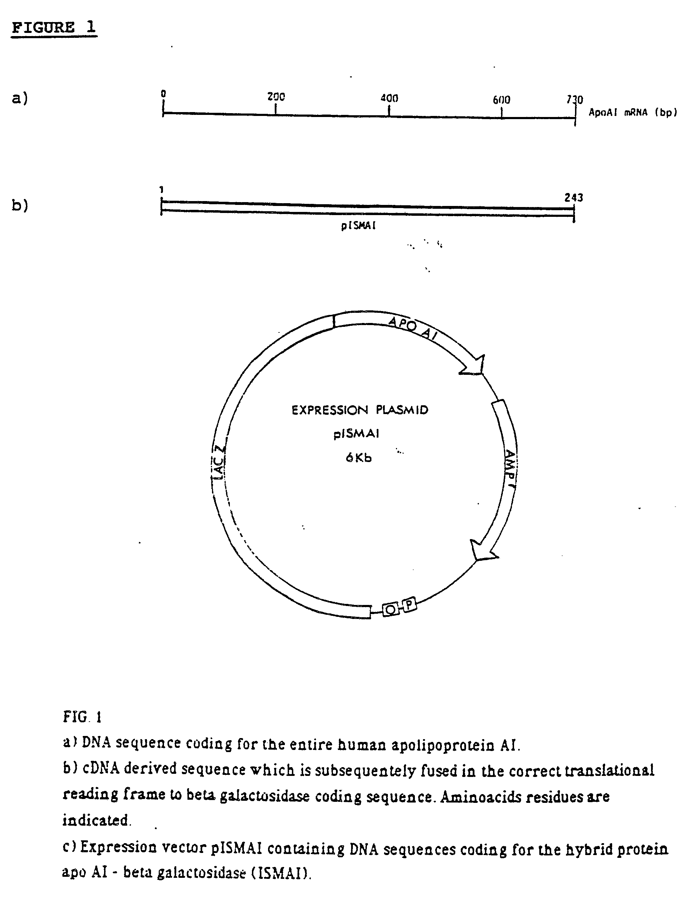

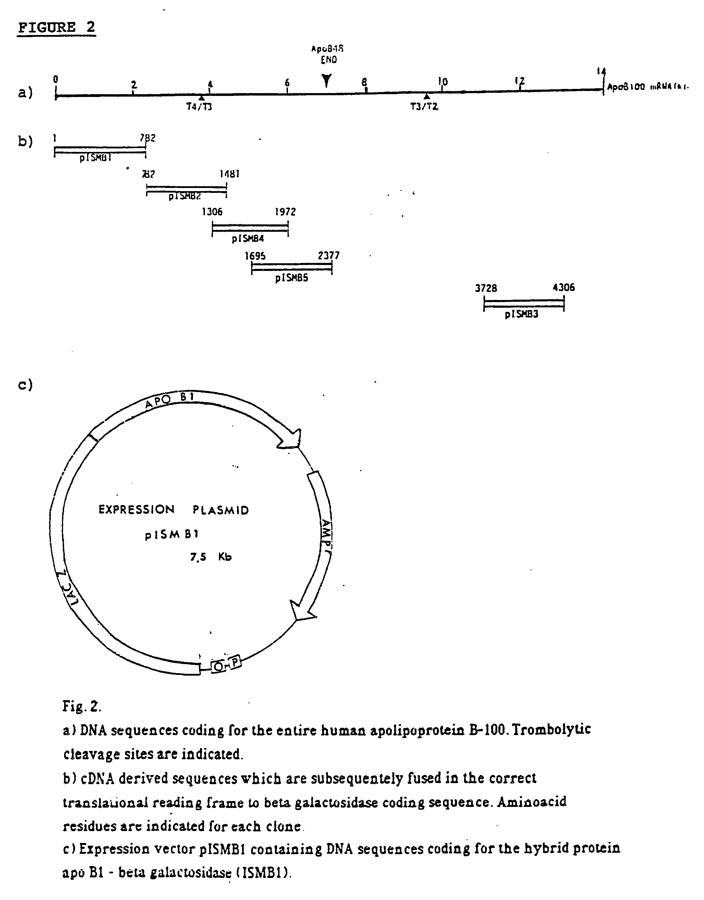

Plasmid pISMAI (ISMAI) was made in the same way as pURAI and the contruction of pISMAI is shown in Figure 1. Using the same process apolipoprotein B-100 DNA coding sequences derived from cDNA clones pABF and pXH2 (Knott et al, Nature (1986) 323 p. 734) and pB4 (Shoulders et al Atherosclerosis (1985) 58 p.277) were cloned in the appropriate expression vectors to give plasmids pISMB1, pISMB2, pISMB3, pISMB4 and pISMB5 as indicated in Figure 2. Only the hybrid protein expressed by pISMB1 (ISMB1) is immunologically reactive with the apo B monoclonal antibody used, as judged by Western Blotting.

## Fermentation Of Hybrid Proteins ISMAI And ISMB1

A single E. coli colony is introduced in one ml. of sterile LB Broth (10 g/l Bacto Tryptone, 5 g/l Bacto Yeast Extract and 10 g/l NaCl) containing ampicillin (100 μg/ml) and grown at 37°C for six hours.

Then this preculture is diluted in 100 ml of fresh sterile LB Broth + ampicillin 100 μg/ml and grown overnight at 37°C on orbital platform. This second preculture is diluted in 10 liters of LB Broth + ampicillin 100 μg/ml sterilized in situ by surriscaldated steam, supplemental with 1 ml of antifoam, in fermentor (Chemap).

Bacteria are allowed to grow at 37°C until the turbiditiy of the medium, measured with the Fundalux apparatus indicates that the culture is at the late exponential phase (2-3 hours).

At this point 10 ml of 1 M isopropyl-beta-D-thiogalactopyranoside (IPTG-Sigma Chem. Co.) are added to the broth (final 1 mM) to induce the protein synthesis; the growth is carried on for two hours.

Finally the broth is cooled on ice and centrifuged at 4000 rpm for 30 minutes at 4°C in a Sorvall refrigerated centrifuge (rotor HG4L).

The bacterial paste is resuspended and washed in buffer Tris HCl 40 mM, pH 8, Na₂ EDTA 0.5 mM, MgCl₂ 1 mM, DTT 1 mM, PMSF 10 mg/l, then centrifuged in Falcon tubes and stored at -80°C.

## Purification: ISMAI

The cell paste is resuspended in 10 ml/g cells of Buffer A (0.1 M Tris HCl. pH7.8 1 mM MgCl₂, 0.5 mM EDTA 0.1 M NaCl containing 0.1 M L-Arginine hydrochloride, 10 mg/l PMSF and 70 mM 2-mercaptoethanol freshly added).

Lysozyme is added to a final concentration of 0.5 mg/ml and the mixture is allowed to stand for 20 minutes at 0°C. Then the cells are disrupted by sonication at 80 Watt for 5 minutes (one minute periods) on ice.

The raw extract is centrifuged at 10000 x g for 10 minutes at 4°C the supernatant is discarded while the pellet is resuspended in the same volume of Extraction Buffer (Buffer A containing 70 mM 2-mercaptoethanol and 0.5% Triton X-100).

The suspension is left in slight agitation at 4°C for 30 minutes then is centrifuged at 10000 x g 10 minutes at 4°C the pellet is discarded and the contaminating proteins are precipitated by slow addition of 114 g/l of anhydrous ammonium sulfate (20% saturation) to the supernatant. The suspension is allowed to equilibrate for two hours at 4°C and then centrifuged at 11000 x g for 15 minutes at 4°C. The pellet is discarded and the supernatant is dialyzed against 2 liters of sodium phosphate buffer 100 mM pH7. The aggregates are removed by centrifugation and the supernatant is divided in small aliquots and lyophilized with 1% glycine and 1% maltose.

## Purification: ISMB1

The cell paste is resuspended in 10 ml/g cells of Sonication buffer (Buffer A 0.1 M Tris HCl pH 7.8, 1 mM MgCl₂ 0.5 mM EDTA 0.1 M NaCl containing 0.1 M L-Arginine hydrochloride, 10 mg/l PMSF, 70 mM 2-mercaptoethanol freshly added)

Lysozyme is added to a final concentration of 0.5 mg/ml and the mixture is allowed to stand for 20 minutes at 0°C.

Then the cells are disrupted by sonication at 80 Watt for 5 minutes (one minute periods) on ice.

The raw extract is centrifuged at 10000 x g for 10 minutes at 4°C the supernatant is discarded, while the pellet is resuspended in the same volume of Extraction buffer (Buffer A, containing 70 mM 2-mercaptoethanol and 0.5% Triton X-100).

The suspension is left in slight agitation at 4°C for 30 minutes, then is centrifuged at 10000 x g for 10 minutes at 4°C; the supernatant is discarded and the pellet is resuspended in 2.5 ml/g cells or Urea buffer - (Buffer A containing 6 M Urea and 70 mM 2-mercaptoethanol).

This suspension is incubated at 4°C for 30 minutes on slow agitation, then centrifuged at 11000 x g for 15 minutes at 4°C.

The pellet is discarded; the supernatant is diluted twofold with Buffer A and dialyzed for 16-18 hours against two liters of the same buffer containing PMSF 5 mg/l and glutathione reduced 1.25 mM and oxidized 0.25 mM. After dialysis the suspension is centrifuged to remove all insoluble aggregates, then it is divided in small aliquots and lyophilized with 1% glycine and 1% maltose.

## Recovery Of Fermentation And Purification Of Hybrid Proteins ISMA And ISMB1

About 50 g cells (wet weight) are usually obtained from a standard culture of ISMA and ISMB1 recombinant E.coli strains in a 10 liter fermentor.

Through purification processes it is possible to obtain about 1g of hybrid proteins per fermentation as 0.5 mg freeze-dried aliquots which are resuspended in PBS to give a suspension having protein concentration 1 mg/ml for ISMAI and 2 mg/ml for ISMB1.

It must be pointed out that these suspensions are not homogeneous and the hybrid proteins represent about 80% for ISMAI and about 60% for ISMB1.

Yet, no further purification step is needed for their use in the assay. For each determination (samples and standard curve) about 5 µg of hybrid protein are required; therefore, from each fermentation, hybrid protein for about 200,000 determinations is obtained.

Usually about 10 determinations in 100 are used for the standard curve and the blanks; therefore, more precisely 180,000 single determinations or 90,000 determinations in duplicate per fermentation are possible.

## Monoclonal Antibodies against Apolipoprotein AI or Apolipoprotein B

The immunoassay method of the invention preferably requires monoclonal antibodies (MAbs) with similar affinity for the hybrid protein produced and the corresponding natural plasma apolipoprotein. Various methods are available for producing monoclonal antibodies fulfilling this requirement, see, for example, Galfre and Milstein, "Preparation of Monoclonal Antibodies, Strategies and Procedures", Methods in Enzymology (1981), 73 pp 1-46, and Reading C.L., J. Immunol. Methods (1982), 53 pp 261-291. Commercially available MAbs may be used if they are sufficiently specific. If no MAb is commercially available, a MAb with the desired properties may be obtained in the following way.

A mouse is immunised with natural antigen (apolipoprotein AI) followed by a boost with the hybrid protein (or the proteins may be administered in the reverse order). Spleen cells from the immunized mouse are then fused with cells of a suitable mouse myeloma cell line in the usual way. The library of fused cells obtained is then screened for MAbs against both ApoAI and the hybrid protein, using an ELISA assay.

Carrying out this process using apolipoprotein B instead of apolipoprotein AI enables a monoclonal antibody against apolipoprotein B to be produced. Since the assay method is particularly useful in indicating the relative amounts of apo AI and apo B, a monoclonal antibody should preferably be chosen which is specific to one of the apolipoproteins but shows no cross-reactivity with the other.

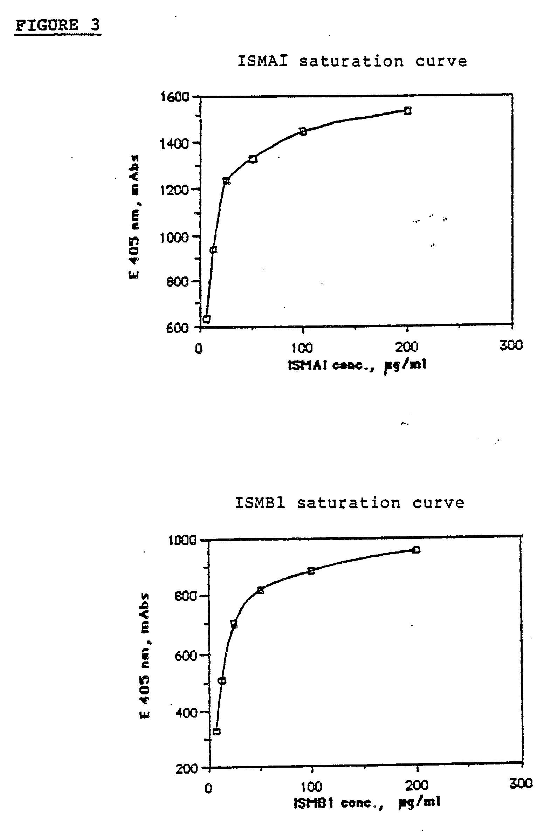

The reaction kinetics of the hybrid proteins ISMAI and ISMB1, in the formation of antigen-antibody complexes, are shown by the saturation curves in Figure 3. These results were obtained by carrying out the following:

- Coating of 96 microwells plate with monoclonal antibodies anti apo AI or anti apo B diluted 1:100 in 0.05 M sodium bicarbonate buffer pH 9.5 at 37°C for 18 hours.
- washing steps with 0.02% Tween-20
- 1% BSA saturation for 1 hour at room temperature
- ISMAI or ISMB1 dilution in PBS
- transfer to the plate of each dilution sample in duplicate (50 µg/well)
- incubation for 1 hour at 37°C for ISMAI and 1/2 hour at 37°C for ISMB1
- washing steps with 0.02% Tween-20

- addition of the beta galactosidase chromogen substrate, ONPG (75mg/100ml), 100μl/well
- incubation at 37°C for 15 min
- addition of the enzyme stop solution, sodium carbonate 1M 100μl/well
- absorbance measure at 405 nm.

Typical saturation curves are obtained for both proteins when the microplates are coated with the corresponding monoclonal antibodies diluted to a final concentration of 10 μg/ml.

A similar result is obtained when ¹²⁵I HDI and ¹²⁵I LDL are employed in the place of ISMAI and ISMB1, respectively.

This comparison shows that the coated monoclonal antibodies are able to react with both the apolipoprotein part of the hybrid protein and the corresponding natural apolipoprotein.

These data are confirmed by the competition assay, between the serum apolipoprotein and the corresponding fusion protein, for the binding to the coated monolconal antibody.

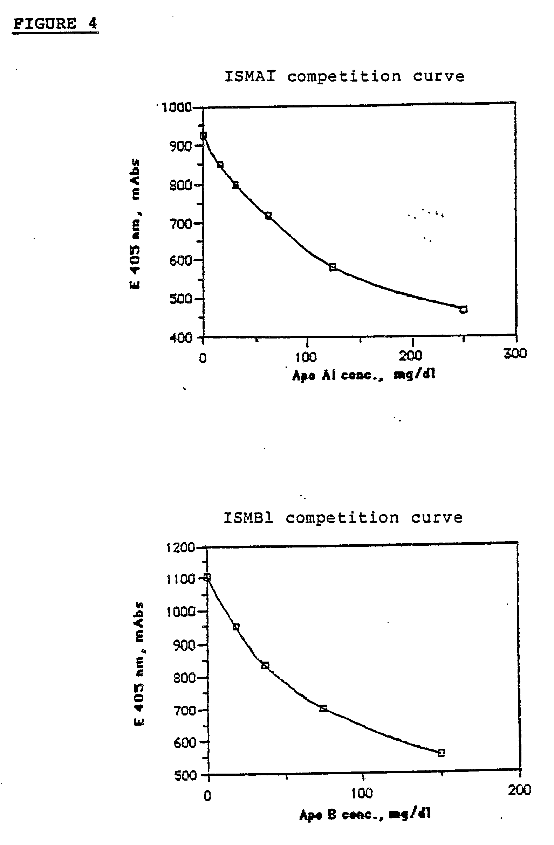

Typical competition curves for apoAI and apo B are shown in Figure 4. These results were obtained by carrying out the following:
- coating of a 96 microwells plate with monoclonal antibodies diluted 1:100 in 0.05 M sodium bicarbonate buffer, pH 9.5 at 37°C for 1 hour
- washing steps with 0.02% Tween-20
- 1% BSA saturation for 1 hour at room temperature
- apo AI or apo B standard serum dilution in PBS and addition of a saturating amount of ISMAI (12.5 μg/ml) or ISMB1 (50 g/ml)
- transfer to the plate of each dilution sample in duplicate (50 l/well)
- incubation at 37°C for 1 hour of ISMAI or 30 minutes for ISMB1
- washing steps with 0.02% Tween-20
- addition of the beta- galactosidase chromogen substrate, ONPG (75mg/100ml), 100μl/well
- incubation at 37°C for 15 minutes
- addition of the enzyme stop solution, sodium carbonate 1M 100 μl/well
- absorbance measure at 405 nm.

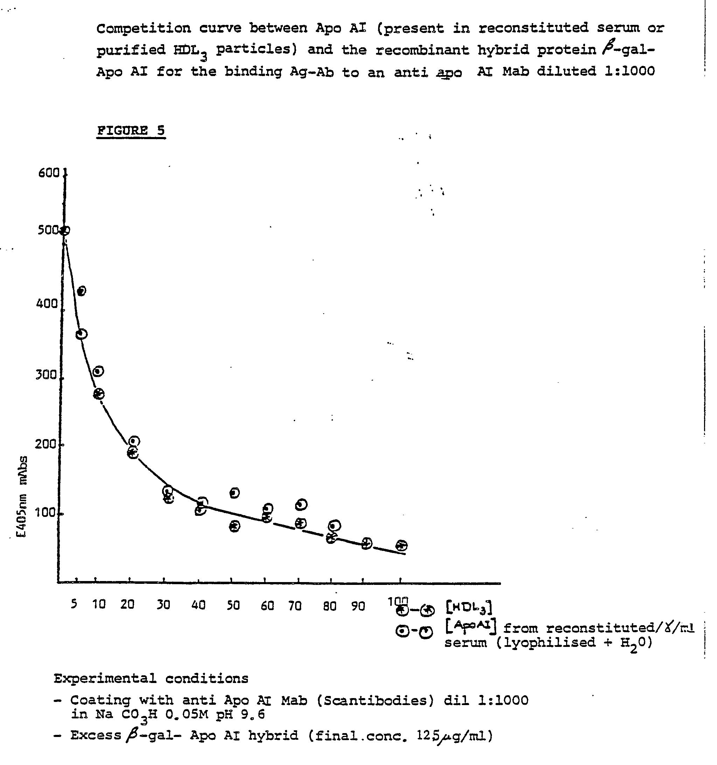

Figure 5 shows the competition curve when anti apo AI antibodies are diluted 1:1000 rather than 1:100. The optimal value of the monoclonal antibodies concentration was tested in a range of 100 μg/ml - 0.1 μg/ml and the best saturation and competition curves were obtained when a concentration of 10 μg/ml, for both monoclonal antibodies anti apoAI and anti apoB, was employed using, as coating buffer, sodium bicarbonate 0.05 M, pH 9.5.

The formation of antigen-antibody complexes linearly increases in absorbance within an hour for ISMAI and 30 minutes for ISMB1. Hence, these reaction times were used for the competition and saturation procedures.

Experimental procedure for the assay

Monoclonal antibodies against apolipoprotein AI (apo AI) were diluted according to the needs of the assay between 100 and 1000 times in 0.05M sodium bicarbonate pH9.6. They were adsorbed on an ELISA plate at 50 μl/well for 2 hours at room temperature or at 4°C overnight. After adsorption, the wells were washed with 0.05% Tween 20 in PBS (0.5M, pH 7.2). The wells were saturated with 1% BSA in PBS for 1 hour at room temperature to reduce background due to non-specific binding. This was followed by 2 washes with 0.05% Tween 20 and 1 wash with PBS. In parallel the sample (e.g. serum, plasma or blood) was diluted 50 times with PBS and 100 μl of the dilution were mixed with an equal volume of the β-gal-apoAI hybrid protein (at 70 μg/ml) obtained from pURAI. In this condition, the β-gal-apo AI protein present is enough to saturate all the monoclonal antibody molecules adsorbed to the plastic (Fig.3.). Using 50 μl/well the competition reaction between the serum apolipoprotein and the β-gal-apoAI recombinant protein for the monoclonal antibody is carried out for 1 hour at 37°C. This is followed up by 3 washes (at 200 μl/well) with 0.05% Tween 20 in PBS and a final wash with PBS.

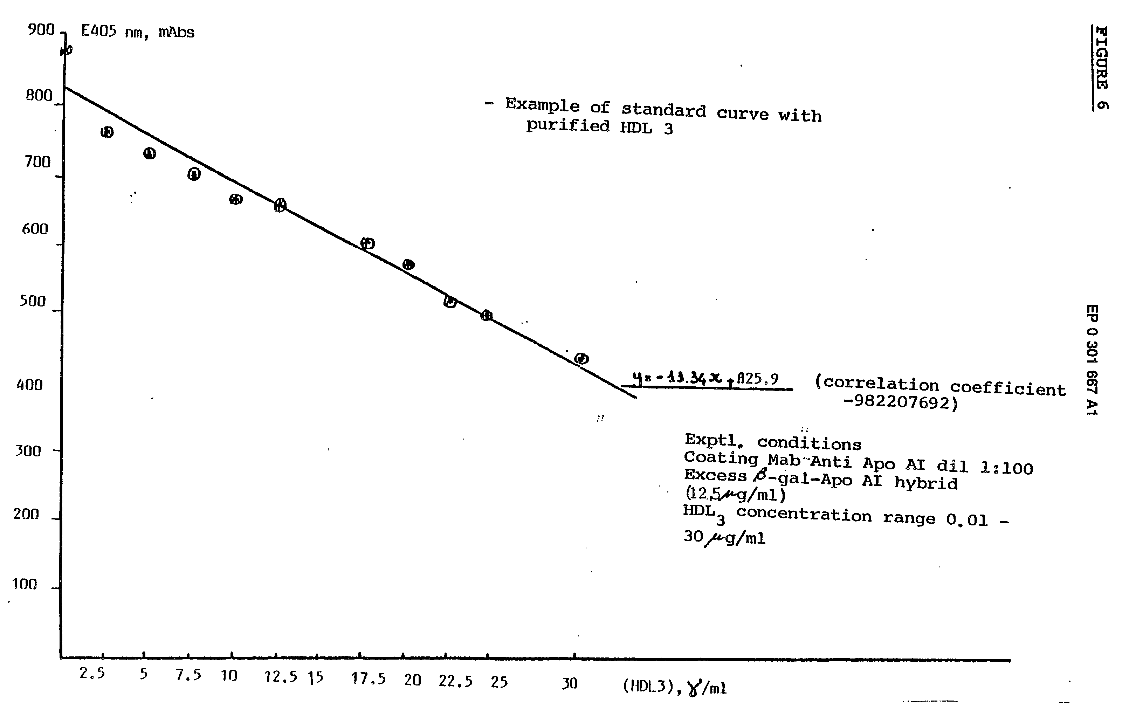

The β-galactosidase activity attached to the MAb via the apoAI was determined as follows:- The substrate for the enzyme (ONPG, o-nitrophenyl-D-galactopyranoside) was dissolved at a concentration of 750 mg/L in Na Phosphate buffer, containing 1 mM $MgCl_2$ at pH7. This solution we made 0.1 M with respect of mercaptoethanol and this incubation mix (100 μl) was added to the microtiter wells and incubated at 37°C for 15 minutes. At this point, 100 μl of stop solution ($Na_2CO_3$.1M) was added and the colorimetric extinction of the solution in the wells was read in a Multiscan Titertek spectrophotometer at 405 nm. From the extinction values expressed in milli absorbance (mAbs) units, the apolipoprotein concentration can be read from a standard curve. The apoAI concentration is inversely proportional to the extinction because a high amount of β-gal-apoAI hybrid retained means a low concentration of apoAI in the serum. Fig.6 shows an example of standard curve obtained with purified HDL3, i.e. high density lipoprotein 3 obtained by zonal ultracentrifugation, see Batsch et al., J. Biological Chem. (1980) 255, pp 3178-3185 and Groot et al, J. Lipid Res. (1982), 23 pp 1342-1353.

The samples were prepared as follows:

| Serum, plasma or whole blood sample | 20μl | 40μl |
|---|---|---|
| PBS | +980μl | +960μl |
| | 1ml of dilution A | 1ml of dilution B |
| | 100μl | 100μl |
| β-gal-apoAI hybrid (25μg/ml) | 100μl | 100μl |
| | 200μl A | 200μl B |
| Final dilution sample | 1:100 | 1:50 |
| Final concentration: β-gal-apoAI hybrid | 12,5μg/ml | 12,5μg/ml |

The apoAI levels were measured in the same individual using as starting material serum, plasma or whole blood. The determinations were repeated with identical results after aliquots of the samples had been stored at 4°C, -20°C and -80°C. The results obtained were as follows:-

| Sample | ApoAI, mg/dl | | |
|---|---|---|---|
| | Serum | Plasma | Blood |
| A | 206 | 202 | 190 |
| B | 120 | 130 | 125 |

Serum apoAI levels were also determined in different individuals, and the new method was compared with the immunodiffusion method (Apoplate-Daichii) currently in clinical use, (based on Mancini et al, Immunochemistry (1965) 2 235 "Quantitation of Antigens by radial immunodiffusion"; see also Carlson et al, Clin. Chem. Acta (1982) 124 pp 163-178 and Lee et al., Atherosclerosis (1974) 19 pp 501-520)

| Sample | ApoAI mg/dl | |
|---|---|---|
| | New method | Immunodiffusion Daichii |
| 1 | 97.5 | 112 |
| 2 | 133.5 | 145 |
| 3 | 109.0 | 139 |
| 4 | 103.0 | 88 |
| 5 | 127 | 132 |
| 6 | 152.0 | 132 |
| 7 | 157.0 | 132 |
| 8 | 134.0 | 132 |
| 9 | 136 | 108 |
| 10 | 131 | 121 |
| 11 | 134 | 108 |
| | VA = 128.5 | VA = 122.6 |

It should be noted that with the Daichii method, only a visual estimation or a densitometer aid estimation is possible. The methods may be compared as follows:-

| | This Invention | Daichii |
|---|---|---|
| Specific | + + + | + + |
| Sensitivity | (0.01 → 1.5γ/well) | (3→ 12γ/well) |
| | + + + + | + |
| Reproducibility | + + + | + |
| Cost | low | high |
| Simplicity of operation | + + + | + |
| Time required for test | 4 h | 48 h |
| Automation | + + + | + |
| Availability of standards | Bacterial fermentation or eukaryotic cell production | Human plasma fractionation |
| Versatility and potential for further development | + + + | + |

## Example: apo AI - apo B determination in whole blood, serum and plasma

### Samples preparation

Blood drawn from normal or dyslipidemic patients, after fasting,were collected in 1 mM EDTA. Plasma and serum samples were prepared following standard methods.

Assay procedure was performed on total blood, plasma and serum within 24 hours storing the samples at 4°C.

For apo AI and apo B determination the samples were diluted in 1:100 mPBS to a final volume of 2 ml.

### Assay Procedure

96 wells plates were coated with 50 μl/well of specific monoclonal antibodies diluted 1:100 in sodium bicarbonate buffer 0.05 M pH 9.5 to a final concentration of 0.5 μg/well.

After 18 hours incubation at 37°C plates were washed twice with a 0.02% Tween-20 solution in PBS.

Plates were saturated with a 1% Bovine Serum Albumin solution PBS at room temperature for 1 hour then three washing steps were performed with a 0.02% Tween-20 solution in PBS.

200 μl of each sample were transferred to a new tube and 10 μl of hybrid protein (ISMAI, 1 mg/ml; ISMB1, 2mg/ml), diluted 1:2 in PBS were added to each sample and to each concentration point of the calibration curve. 50μl of these samples were dispensed in duplicate in each well and incubated at 37°C for an hour (ISMAI) or 30 minutes (ISMB1). Wells were washed three times with 0.02% Tween-20 solution in PBS.

Beta galactosidase activity was assayed by adding 100 μl/well of the chromogen substrate O-nitro-phenyl-galactosidase (ONPG), at a concentration of 75 mg/100 ml in 0.1 M sodium phosphate buffer, pH7, containing 1 mM $MgCl_2$.

Incubation was at 37°C for 15 minutes and the enzymatic reaction was stopped adding 100 μl/well of 1M $Na_2CO_3$.

Absorbances were measured at 405 nm with a microplate reader.

The apo AI levels were measured in the same individual using, as starting merial, serum, plasma or whole blood.

| | apo AI, mg/dl | | |
|---|---|---|---|
| Sample | Serum | Plasma | Blood |
| A | 206 | 202 | 190 |
| B | 120 | 130 | 124 |
| C | 212 | 171 | 195 |
| | - | apo B, mg/dl | |
| Sample | Serum | Plasma | Blood |
| A | 74.5 | 70 | 77 |
| B | 63 | 70 | 73 |
| C | 72 | 74.4 | 81 |

In this determination, obtained values are corrected to a dilution factor referred to the sample volume.

Serum 1 ml Apo AI x 1 - Apo B x 1

Plasma 1.2 ml Apo AI x 1.2 - Apo B x 1.2

Blood 2 ml Apo AI x 2 - Apo B x 2

## Intra-Assay Coefficient Of Variation (CV %) In The Determination Of Apo AI

Apo AI determnation in one sample (sample B) assayed 84 times on one microplate

```
apo AI      101 121 123 108 114 107 109 120 120 110 118 112
mg/dl       106 114 120 120 121 112 115 118 132 134 118 124
            115 120 117 118 137 140 113 115 123 112 119 127
            128 116 123 116 107 120 121 121 138 121 124 112
            123 118 109 124 123 120 130 123 123 118 125 115
            108 124 114 127 117 111 106 118 117 116 130 121
            130 141 116 118 104 109 120 115 114 114 109 120
```

mean value: 118.69 mg/dl

S.D: 7.99

intra-assay C.V%: 6.7

## APO AI determination in three samples (A,B,C) dispensed on the microplate according to the following order:

```
A A C C B B B B C C C C    130,132,59,63,125,124,105,108,70,60,68,62
A A A A B B A A C C B B    167,172,173,179,130,124,145,160,70,74,123,12
B B A A                    118,117,148,150
```

Sample A mean value: 155.65 mg/dl

S.D: 17.18

Intra-assay C.V%: 11.04

Sample B mean value: 120 mg/dl

S.D: 8

Intra-assay C.V% 6.7


Sample C mean value: 65.7 mg/dl

S.D: 5.4

Intra-assay C.V%: 8.3


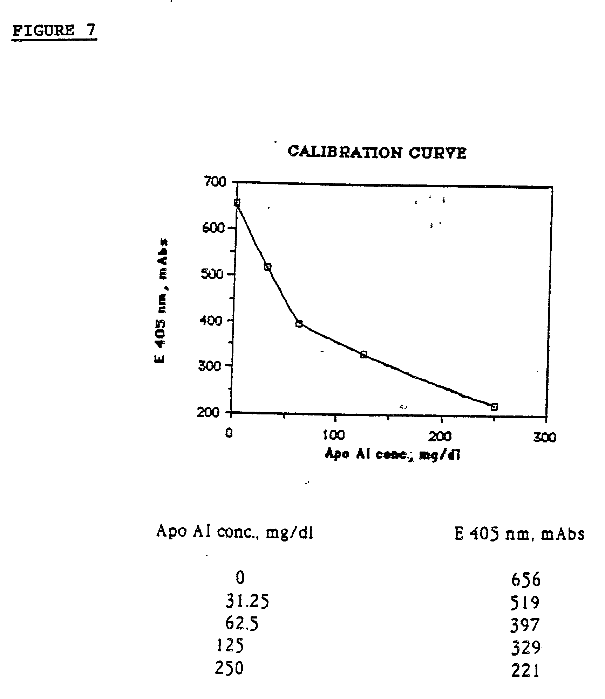

All apo AI values are calculated usng the CALIBRATION CURVE of Figure 7.


Intra-Assay Coefficient Of Variation (CV%) In The Determination Of Apo B

Following the same procedure to determine the intra-assay coefficient of variation (CV%) of Apo AI, the mean value found of Apo B concentration (n = 84) was 89.25 mg/dl and the intra-assay C.V.% was 8.8.


Inter-Assay Coefficient Of Variartion (CV%) In The Determnation Of Apo AI And Apo B

APO AI inter-assay CV% = 9.4
APO B inter assay CV% = 9.7
N.B. For both inter-assay CV% determinations the same samples were tested in 4 different times in double.


Determination Of Apo AI And Apo B Assay Sensitivity

Using serial dilutions of Apo AI - Apo B standard serum the maximum sensitivity value found for Apo AI determination is 1μg/ml and the minimal sample volume necessary to perform one determination in double is 2μl.

For Apo B determination the maximum sensitivity value is 1.2 μg/ml and, also in this case, the minimal sample volume for one determination in double is 2μl.

The same procedure can be used for the determination of other apolipoproteins (AII, AIV, CI, CII, CIII, D, E). A computation of their plasma levels and ratios is a more valuable indicator of cardiovascular risk than the current hyperlipidemic indexes.

The method according to the present invention can be named: RIECA (Recombinant Immuno-Enzymatic Competition Assay).


**Claims**

1. A method of detection or estimation of an apolipoprotein in a sample which comprises contacting a solid support having an antibody to said apolipoprotein bound thereto with the sample containing said apolipoprotein and with fused protein as defined herein comprising at least an antigenic part of said apolipoprotein and a label protein and then observing or measuring the label protein either bound or not bound to the said support.

2. A method according to claim 1 in which the antigenic sequence of the apolipoprotein in the fused protein comprises more than 150 amino acids.

3. A method according to claim 1 or 2 in which the apolipoprotein is apolipoprotein AI.

4. A method according to claim 1 or 2 in which the apolipoprotein is apolipoprotein B.

5. A method according to claim 1 or 2 in which the solid support has bound thereto antibodies to both apolipoprotein AI and apolipoprotein B, and the fused protein comprises a first fused protein comprising an antigenic part of apolipoprotein AI and a first label protein, and a second fused protein comprising an antigenic part of apolipoprotein B and a second label protein which is different from the first label protein.

6. A method according to any one of the preceding claims in which the fused protein is coded for by a gene which comprises a bacterial gene and a eukaryotic gene.

7. A method according to claim 6 in which the bacterial gene codes for the label protein and the eukaryotic gene codes for the apolipoprotein to be detected.

8. A method acording to any one of the preceding claims in which the label protein is peroxidase.

9. A test kit for operation of the method according to any one of the preceding claims comprising a solid support having an antibody bound thereto and the hybrid protein.

## FIGURE 1

FIG. 1

a) DNA sequence coding for the entire human apolipoprotein AI.

b) cDNA derived sequence which is subsequently fused in the correct translational reading frame to beta galactosidase coding sequence. Aminoacids residues are indicated.

c) Expression vector pISMAI containing DNA sequences coding for the hybrid protein apo AI - beta galactosidase (ISMAI).

## FIGURE 2

Fig. 2.

a) DNA sequences coding for the entire human apolipoprotein B-100. Trombolytic cleavage sites are indicated.

b) cDNA derived sequences which are subsequentely fused in the correct translational reading frame to beta galactosidase coding sequence. Aminoacid residues are indicated for each clone.

c) Expression vector pISMB1 containing DNA sequences coding for the hybrid protein apo B1 - beta galactosidase (ISMB1).

## FIGURE 3

ISMAI saturation curve

ISMB1 saturation curve

## FIGURE 4

### ISMAI competition curve

### ISMB1 competition curve

Competition curve between Apo AI (present in reconstituted serum or purified HDL₃ particles) and the recombinant hybrid protein β-gal-Apo AI for the binding Ag-Ab to an anti apo AI Mab diluted 1:1000

FIGURE 5

Experimental conditions

- Coating with anti Apo AI Mab (Scantibodies) dil 1:1000
  in Na CO₃H 0.05M pH 9.6
- Excess β-gal- Apo AI hybrid (final.conc. 125μg/ml)

FIGURE 6

EP 0 301 667 A1

**FIGURE 7**

### CALIBRATION CURVE

| Apo AI conc., mg/dl | E 405 nm, mAbs |
|---|---|
| 0 | 656 |
| 31.25 | 519 |
| 62.5 | 397 |
| 125 | 329 |
| 250 | 221 |

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,D | WO-A-8 606 742 (CALIFORNIA BIOTECHNOLOGY)<br>* Page 3, line 9 – page 5, line 15; page 8, line 29 – page 9, line 4; page 10, line 27 – page 11, line 32; page 12, line 22 – page 14, line 14; page 29, lines 21-28; page 30, line 8 – page 31, line 4; page 32, lines 1-9 *<br>--- | 1,3-9 | G 01 N 33/92<br>G 01 N 33/535 |
| X | WO-A-8 604 144 (INTERNATIONAL GENETIC ENGINEERING)<br>* Page 5, lines 15-25; page 6, lines 1-28; page 9, lines 8-32; page 10, lines 8-11; page 16, line 12 – page 18, lines 6,10 – page 20, line 25; page 35, example 4; page 36, example 5; page 37, lines 2-15; page 42, line 25 – page 43, line 11 *<br>--- | 1,3-5,8 ,9 | |
| X | WO-A-8 002 460 (LABORATORIES GOELLA)<br>* Page 1, lines 6-19; page 10, example 3; page 15, line 2 – page 16, line 35 *<br>--- | 1,3,4,8 ,9 | |
| X,D | WO-A-8 702 061 (BIOTECHNOLOGY RESEARCH PARTNERS LTD)<br>* Page 4, lines 6-16; page 6, lines 10-30; page 20, lines 18-23; page 26, line 24 – page 28, line 34; page 38, lines 4-29; figures 1-5 *<br>--- | 1-7 | TECHNICAL FIELDS SEARCHED (Int. Cl.4)<br><br>G 01 N 33/00 |
| X,P | US-A-4 745 055 (D.B. SCHENK)<br>* Column 5, lines 1-7; column 5, line 62 – column 6, line 47; column 6, line 52 – column 7, line 44 *<br>---      -/- | 1,3-9 | |

The present search report has been drawn up for all claims

| Place of search | Date of completion of the search | Examiner |
|---|---|---|
| THE HAGUE | 05-11-1988 | VAN BOHEMEN C.G. |

EPO FORM 1503 03.82 (P0401)

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| Y | WO-A-8 605 493  (SCRIPPS CLINIC AND RESEARCH FOUNDATION)<br>* Page 5, lines 18-26; page 12, lines 17-34; page 40, lines 10-27 *<br>--- | 1-9 | |
| Y | US-A-4 356 270  (K. ITAKURA)<br>* Column 4, lines 9-52; column 5, line 55 - column 11, line 4 *<br>--- | 1-9 | |
| Y | WO-A-8 602 666  (MICROGENICS CORP.)<br>* Page 11, line 10 - page 12, line 6; page 28, line 21 - page 30, line 22; figure 1 *<br>--- | 1-9 | |
| X | CLINICAL CHEMISTRY, vol. 28, no. 1, January 1982, pages 59-62, Easton, Pennsylv., US; J.-C. FRUCHART et al.: "Simultaneous measurement of plasma apolipoproteins A-1 and B by electroimmunoassay"<br>* Page 59, column 1, lines 1-19 *<br>----- | 3-5 | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 05-11-1988 | VAN BOHEMEN C.G. |